# EUROPEAN PATENT SPECIFICATION

(11) **EP 1 335 719 B1**
(45) Date of publication and mention of the grant of the patent: **19.12.2007**
(21) Application number: 01934672.5
(22) Date of filing: 11.05.2001
(51) Int. Cl.: A61K 31/23, A61P 17/00

(54) **CETYL MYRISTATE AND CETYL PALMITATE FOR TREATING ECZEMA AND/OR PSORIASIS**
CETHYLMYRISTAT UND CETYLPALMITAT ZURBEHANDLUNG VON EKZEMEN UND/ODER PSORIASIS
CETYL MYRISTATE AND CETYL PALMITATE POUR LE TRAITEMENT DE L'ECZEMA ET/OU DU PSORIASIS

(30) Priority: 12.05.2000 NZ 50452500; 28.09.2000 NZ 50722800
(43) Date of publication of application: 20.08.2003
(73) Proprietor: Meracol Corporation Limited, Auckland (NZ)
(72) Inventor: MEAKIN, Timothy David, Auckland (NZ); HEATLEY, Craig Leonard, Auckland (NZ); CADWALLADER, Dianne, Auckland (NZ)
(74) Representative: Baldock, Sharon Claire
(86) International application number: PCT/NZ2001/000085
(87) International publication number: WO 2001/085163

(56) References cited:
- WO-A-00/07627
- WO-A-00/67728
- WO-A-01/85162
- WO-A-99/56733
- WO-A-99/60167
- GB-A- 2 170 407
- GB-A- 2 337 461
- US-A- 5 496 565
- US-A- 5 886 038
- AMEREX CORPORATION: "What is cetyl myristate" INTERNET PUBLICATION, [Online] - 20 January 1999 (1999-01-20) XP002265364 Retrieved from the Internet: <URL:www.hollinet.com/~jwin/Cetyl%20Myrist ate.htm> [retrieved on 2003-12-17]
- AMEREX CORPORATION: "Questions about Cetyl Myristate" INTERNET PUBLICATION, [Online] - 20 January 1999 (1999-01-20) XP002265348 Retrieved from the Internet: <URL:http://www.hollinet.com/~jwin/QuestCe tyl.htm > [retrieved on 2003-12-17]
- WHOLE HEALTH DISCOUNT CENTER: "Myristin" INTERNET PUBLICATION, [Online] - 16 December 2003 (2003-12-16) XP002265368 Retrieved from the Internet: <URL:www.health-pages.com/pain/cm.html> [retrieved on 2003-12-17]

## Description

### TECHNICAL FIELD

The present invention relates to a method of treatment and/or prophylaxis of eczema and psoriasis.

### BACKGROUND

Eczema can be described as an inflammation of the skin where swelling, redness, itching or a burning sensation is present. Sometimes the first inflammation is felt, rather than seen, as it is immediately beneath the skin's surface. Eczema can also be seen as reddened spots, scales, crusts or blisters may also be present, either alone or in combination. It may take a mild form, or be more severe, as in the case of psoriasis.

The present invention has surprisingly determined that the ingestion of cetyl myristate, and particularly cetyl myristate in conjunction with cetyl palmitate, provides an effective treatment of eczema and/or psoriasis.

Cetyl myristate and cetyl palmitate can each be sourced from animals or vegetables. Cetyl myristate is not to be mistaken for cetyl myristoleate which is also a fatty acid derived traditionally from spermaceti by saponification and more recently from the tallow of bovine(s).

Reference is made to US Patent No.4,113,881 where it is disclosed that the administration of an effective amount of cetyl myristoleate to a mammal is useful in inhibiting or relieving the symptoms of inflammatory rheumatoid arthritis in mammals. Also in US 5,569,676 there is disclosure of the use of cetyl myristoleate in the treatment of osteo-arthritis.

It is thought that cetyl myristate has a negligible anti-arthritic activity in laboratory experiments and reference is made to the website www.gcinutrients.com/Newletter.com. However this point is arguable and a product known as cetyl myristate sold by Amerex Corporation of 770 Sycamore Avenue, Suite J148, Vista, CA 92083, USA purports that cetyl myristate is useful for the treatment of arthritis.

Cetyl myristate is derived from the saturated fatty acid, myristic acid. This acid is found in nutmeg butter, in the fats of Myristicaceae, in palm seed fats, milk fats and also sperm whale oil. Reference is made to US 2,481,365 which discloses the preparation of myristic acid from tall-oil fatty acids. It is to be noted that Amerex Corporation source the cetyl myristate used in their products from sunflower oil. See their website at www.hollinet.com.

Cetyl palmitate is derived from the fatty acid, palmitic acid which occurs as the glycerol ester in many oils and fats such as palm oil or Chinese vegetable tallow. A synthetic method of preparation is to react palmitoyl chloride and cetyl alcohol in the presence of magnesium. See the Merck Index, 12th edition at page 336. Reference is also made to US patent 3,169,099 which discloses a biosynthetic method of producing cetyl palmitate.

It is an objection of the present invention to provide a medicament to aid in the treatment and/or prophylaxis of eczema and psoriasis which will provide an alternative to existing treatments or to provide the public with a useful choice.

### DISCLOSURE OF INVENTION

As indicated earlier the present invention is concerned with the treatment and/or prophylaxis of eczema and/or psoriasis reliant upon administration (whether by self administration or otherwise) of either cetyl myristate or cetyl myristate and cetyl palmitate (whether given simultaneously in admixture or not or given serially).

Also described are dosage forms that contain both cetyl myristate and cetyl palmitate and dosage regimes that might use this dosage form.

The invention is the use of (a) cetyl myristate or (b) cetyl myristate and cetyl palmitate in the manufacture of a medicament for a method of treatment and/or a prophylaxis of a mammal for eczema and/or psoriasis in a mammal. Thus, an effective amount of said
(a) cetyl myristate, or
(b) cetyl myristate and cetyl palmitate, is administered.

Preferably said administration is orally of (b) whether as a mixture of both cetyl myristate and cetyl palmitate, or serially.

Preferably the effective amount is of (b).

Preferably said administration is with a mixture of cetyl myristate in conjunction with cetyl palmitate where the cetyl myristate comprises from 50 to 98% w/w of the mixture.

Preferably said effective amount of (a) or (b) is by means of one or more capsules.

In one type of use said mammal is a human being suffering from eczema and the administration is for treatment purposes.

In another type of use said mammal is a human being suffering from psoriasis and the administration is for treatment purposes.

Also described is an oral pharmaceutical composition for treating eczema which comprises or includes both cetyl myristate and cetyl palmitate.

Also described an oral pharmaceutical composition for treating psoriasis which comprises or includes both cetyl myristate and cetyl palmitate.

Preferably said cetyl myristate comprises at least 50% by weight of the composition.

Preferably said composition also includes at least one pharmaceutically acceptable excipient and/or diluent.

Also described is an oral dosage unit effective in the treatment of eczema and/or psoriasis, said dosage unit having a mixture of cetyl myristate and cetyl palmitate.

Preferably in said dosage unit said cetyl myristate in any such mixture comprise from 50 to 98% w/w of the mixture.

Preferably in the dosage use, where the mixture is present, there is from 5 to 400 mg of cetyl myristate and cetyl palmitate.

Preferably the mixture is a capsule.

Preferably said capsule also includes a pharmaceutically acceptable excipient and/or diluent.

Preferably the dosage unit includes silicon dioxide.

Preferably the dosage unit also contains calcium phosphate and/or magnesium oxide.

Preferably the dosage unit also includes additionally at one trace element.

Also described is a liquid composition being also a composition as aforesaid or a dosage unit as aforesaid.

Also described is the use, in the manufacture of oral dosage units for the treatment or prophylaxis of eczema and/or psoriasis in a mammal, of
(a) cetyl myristate, or
(b) a mixture of cetyl myristate and cetyl palmitate.

Also described is the use, in the manufacture of oral dosage units for the treatment or prophylaxis of eczema and/or psoriasis in a mammal, of
(i) cetyl myristate, and
(ii) cetyl palmitate.

We have also noted that the present invention in conjunction with an accelerated wound healing utilising topical composition (for example as disclosed in US 4775291) can supplement effectively the effects thereof with oral dosages of either (a) or (b) as defined above.

The mixture can use cetyl myristate available from a commercial source such as EHP Products Inc., PO Box 20727, Mt Pleasant, SC 29465 or at Amerex Corporation, 770 Sycamore Avenue Suite J148 Vista, California 92083.

The mixture can use cetyl palmitate derived from a source such as, for example, Quimica Croda, S.A. de C.V, Circuito Médicos No.47. Apdo. Postal 71-A Cd. Satélite, 53100 Naucalpan, Edo. de México, México or online at www.butterburandsage.com.

Most ideally however the mixture is synthetised from starting materials utilising the procedures as disclosed in New Zealand Patent Specification No. 332959 which involves reacting both myristic acid and palmitic acid with a cetyl alcohol at an elevated temperature in the presence of at least one acid catalyst and at least one aromatic hydrocarbon. The aromatic hydrocarbon fraction then contains the cetyl myristate and cetyl palmitate from whence it can be crystallised.

This crystallised form can then be ground up, dissolved and mixed with a suitable general pharmacy liquid to be administered to a person. The crystals are usually dissolved in hot water before adding to the pharmacy liquid which is usually a sugar syrup available from most pharmaceutical companies. The liquid is made up to a concentration of 70 w/v.

Alternatively the crystals may be ground up into a powder and combined with magnesium oxide, silicon oxide and fine di-calcium phosphate. This powder can then be transferred into capsules for oral ingestion into the body. The capsules used are VEGICAP^{™} that are non-gelatin containing.

The mode of administration is preferably oral. The dosage unit can be either a swallowable capsule or some alternative (preferably having the active ingredient(s) as a wax-like solid or can be an orally consumable liquid composition (eg; made up with a general pharmacy type carrier such as methyl cellulose)).

Other modes of administration can include transdermal and suppository delivery (the latter being generally contraindicated having regard to the targeted condition).

The administration process involves either orally ingesting capsules or drinking the liquid formulation either on an empty stomach or not. The number of capsules or liquid taken depends on the size and severity of the persons condition. Generally, an adult suffering from Eczema and/or psoriasis is advised to take at least 4 capsules 3 times daily of a dosage unit as described in the invention, whereas for a child this is reduced to half or less. The dosage may be increased or decreased depending on whether the symptoms begin to clear up.

Similarly for the liquid formulation where an amount of liquid equivalent to at least 4 capsules is prescribed which is to be taken 3 times daily. That is 4200 mg of cetyl myristate or the mixture of cetyl myristate and cetyl palmitate.

### Drawings

The present invention will hereafter be described with reference to both trial examples to the accompanying figures which give an indication of the condition of the patient prior to taking the dosage unit of either the capsule or liquid form as described in the present invention and the subsequent condition of the patient after taking the dosage unit as described in the present invention.

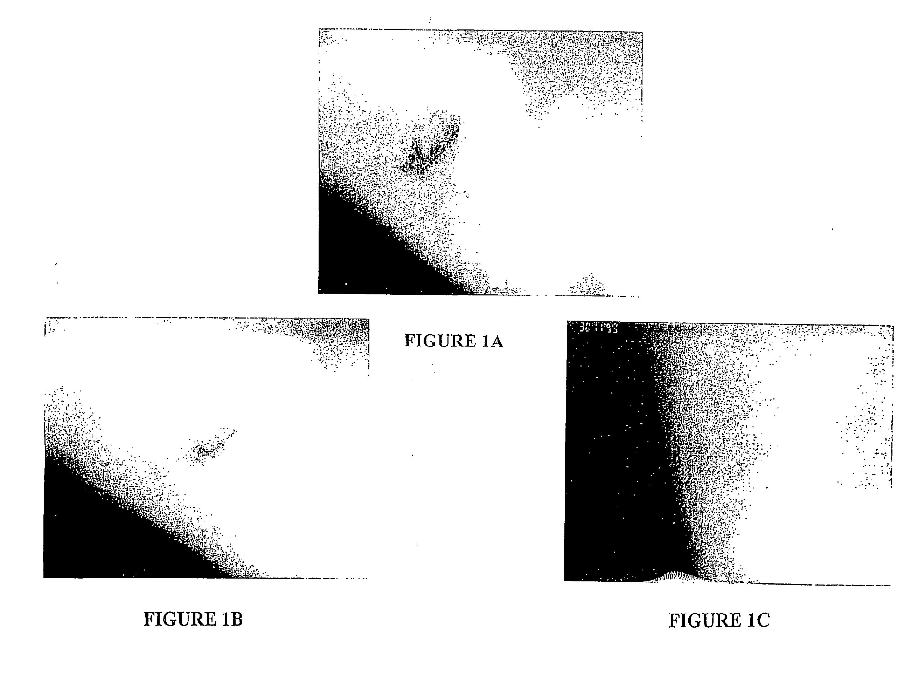

**Figure 1A** shows the condition of a flexural point of patient 1 before taking the liquid dosage as described in the invention. The eczema has inflamed and swollen the skin tissue to a point that a lesion has formed with a crust forming around the edges of the lesion as the body is trying to repair the skin. The patient was then prescribed the liquid dosage as described in the invention at a dosage rate of 5 mls 3 times daily.
**Figure 1B** shows the same flexural point one week after taking the liquid dosage as prescribed. The inflammation and swelling previously present one week before has now diminished and the lesion is now healing over.
**Figure 1C** shows the same flexural point two weeks after taking the liquid dosage as prescribed. The lesion has now completely healed, there is no longer any inflammation or swelling and the skin where the lesion had been is now clear of any eczema.

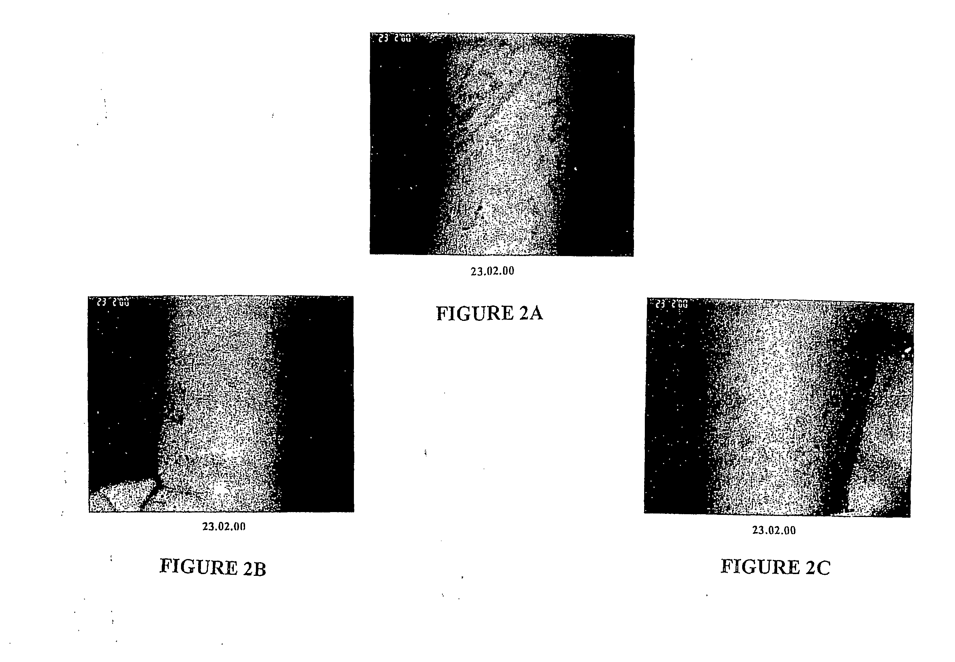

**Figure 2A-2C** shows the condition of patient 2's upper arm, wrist and leg, respectively, before taking the liquid dosage as described in the invention. The eczema has inflamed and swollen the skin tissue to a point that multiple lesions and spots have formed, this is particularly so at the flexural point as seen in figure 2B. The patient was then prescribed the liquid dosage as described in the invention at a dosage rate of 5 mls 3 times daily,

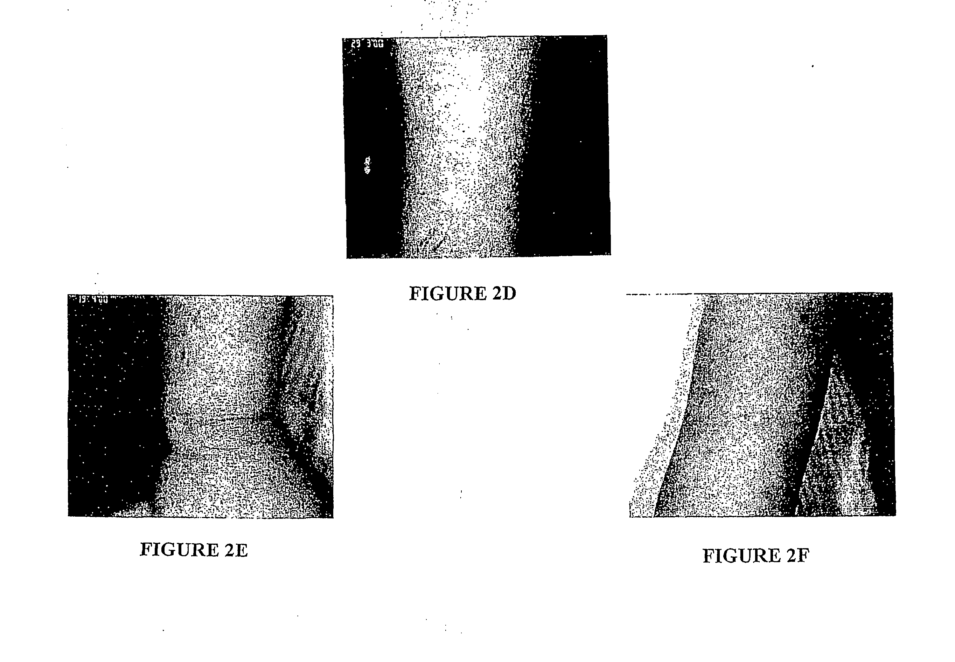

**Figure 2D** shows patients 2's leg 6 days after taking the prescribed dosage as described in this invention. The inflammation and swelling previously present has now decreased where the multiple lesions have started to heal over and have become superficial,
**Figure 2E and 2F** shows patient 2's upper arm and wrist area 27 days after taking the prescribed dosage as described in this invention. The multiple lesions and spots have healed over leaving only the scar tissue from the eczema. The flexural point as shown in figure 3E has healing scabs where previously there had multiple lesions,

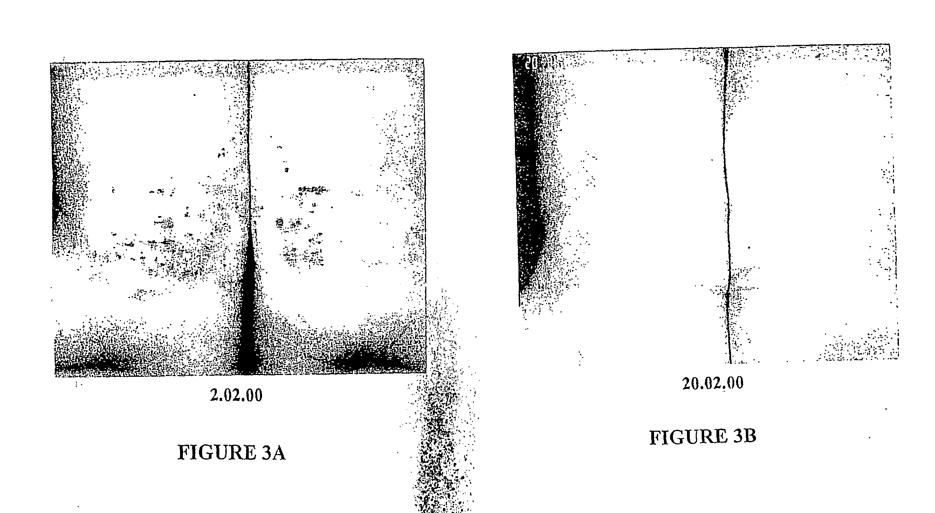

**Figure 3A** shows the condition of the wrists of patient 5 before taking the liquid dosage as described in this invention. The eczema has caused multiple inflamed spots on the skin which have formed a crust over the areas of swelling. The patient was then prescribed the liquid dosage as described in the invention at a dosage rate of 5mls, 3 times a day,
**Figure 3B** shows the condition of the wrists of patient 5, 18 days after taking the dosage as described in this invention. The skin where the eczema had affected has now completely cleared up of the eczema with the skin returning to a normal appearance, without any scars or presence of the customary spots of eczema,

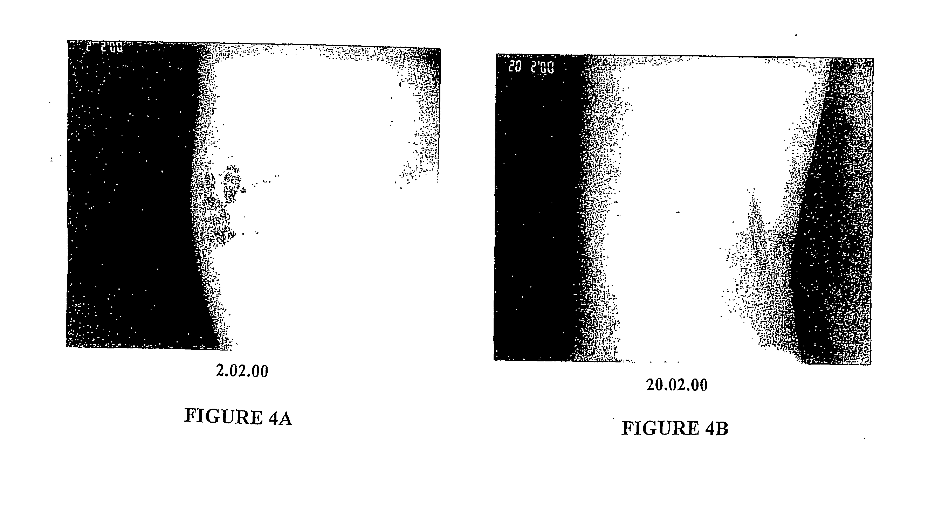

**Figure 4A** shows the condition of a flexural region on the elbow of patient 5 before taking the liquid dosage as described in this invention. The eczema has caused inflamed spots and lesions to form along the creases of the flexural region. The patient was then prescribed the liquid dosage as described in the invention at a dosage rate of 5mls, 3 times a day,
**Figure 4B** shows the condition of the same flexural region as shown in figure 4B after patient 5 has taken the prescribed dosage after 18 days. The eczema has cleared up, the swelling and inflammation has disappeared and the skin has returned to a normal appearance.

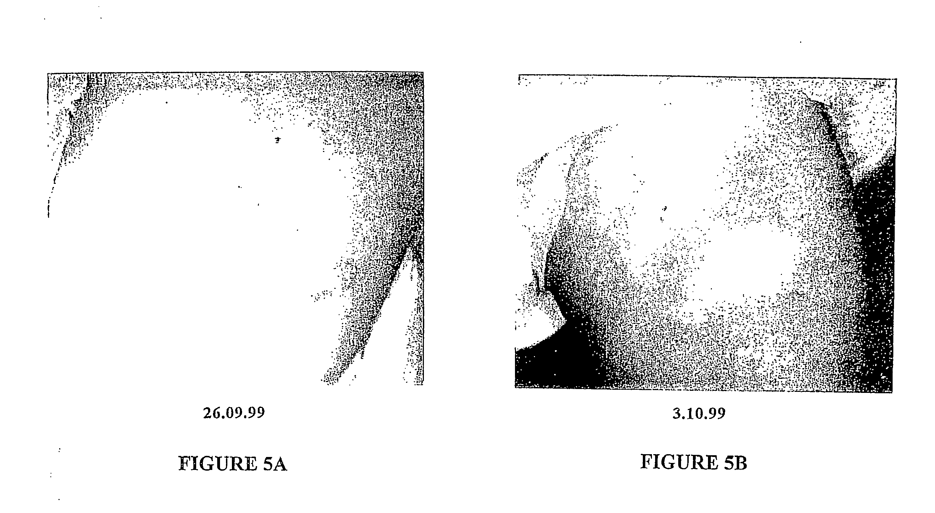

**Figure 5A** shows the condition of the upper thigh of patient 7 before taking the liquid dosage as described in this invention. The eczema has caused splotchy inflamed spots to appear on the skin. The patient was then prescribed the liquid dosage as described in the invention at a dosage rate of 5mls, 3 times a day,
**Figure 5B** shows the condition of the same upper thigh of patient 7 after taking the prescribed dosage for one week. The eczema has almost disappeared with only a few spots remaining that are no longer inflamed,

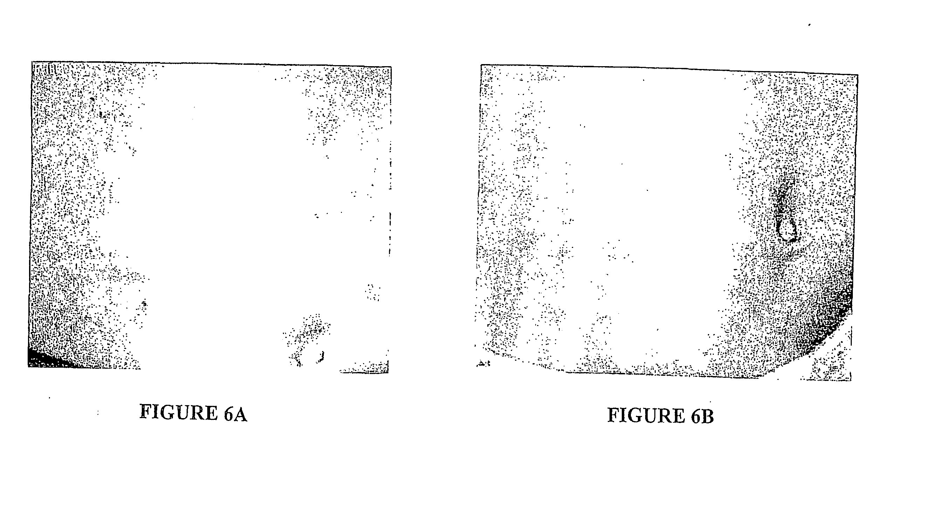

**Figure 6A** shows the condition of patient 9's stomach area before taking the liquid dosage as described in this invention. The eczema has caused patchy spots to appear all over the skin and unlike the previous figures, this eczema is not as inflamed or swollen. The patient was then prescribed the liquid dosage as described in the invention at a dosage rate of 7.5mls, 3 times a day,
**Figure 6B** shows the condition of patient 9's stomach area 19 days after taking the liquid dosage as prescribed. The skin area is now clear of eczema with the skin starting to return to a smooth appearance, however the spots of eczema are still present,

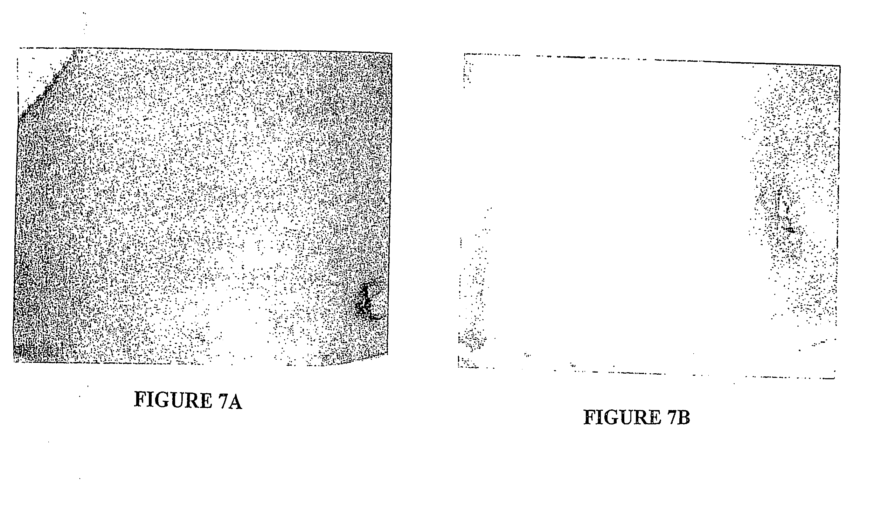

**Figure 7A** shows the condition of patient 8's stomach area before taking the liquid dosage as described in this invention. The eczema has caused inflamed red patchy spots to appear all over the skin. The body has been trying to heal the eczema and so there is also associated tan coloured patchy spots all over the skin as well. The patient was then prescribed the liquid dosage as described in the invention at a dosage rate of 5mls, 3 times a day,
**Figure 7B** shows the condition of patient 8's stomach area 6 months after taking the liquid dosage as prescribed. The eczema has now completely disappeared with the skin returning to a smooth blemish free appearance.

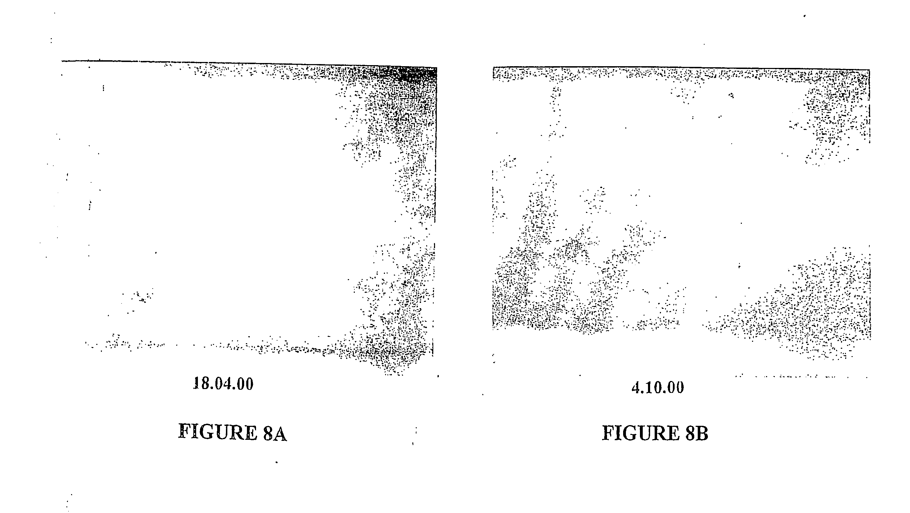

**Figure 8A** shows the condition of patient 10's arm before taking the capsule dosage as described in this invention. The eczema has caused the skin to be scaly and dry with the associated inflammation and swelling of the skin in localised areas. The patient was then prescribed with the capsules of a dosage unit as described in this invention which were taken in groupings of 4 capsules, 3 times a day,
**Figure 8B** shows the condition of the same arm almost 6 months after patient 10 has taken the capsule dosage as prescribed. The skin is no longer dry and scaly. Rather the skin has a sheen that makes the skin appear as though the moisture has been replaced. The inflammation and swelling has decreased although it has not disappeared completely,

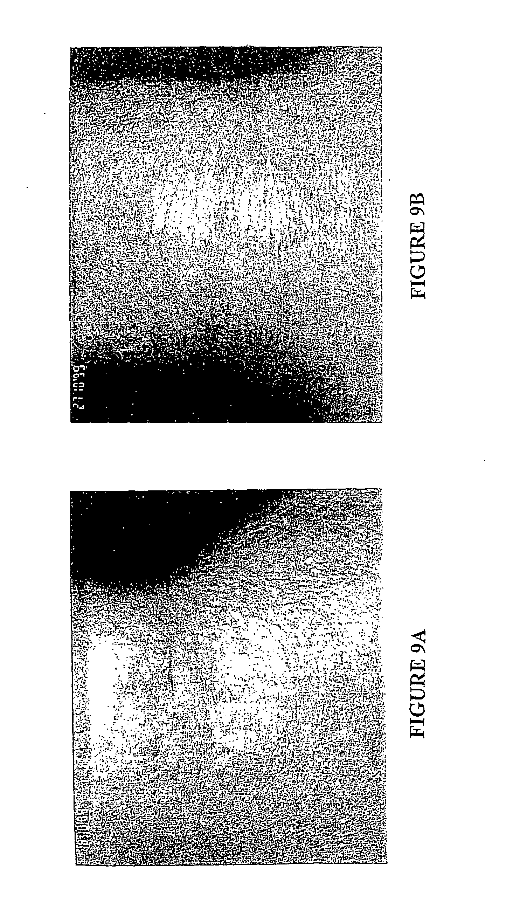

**Figures 9A** shows the condition of patient 4's wrist before taking the liquid dosage as described in this invention. The eczema has caused inflammation and swelling of the skin along the flexural lines between the wrist and hand with small spots and lesions forming. The patient was then prescribed the liquid dosage as described in the invention at a dosage rate of 10mls, 3 times a day,

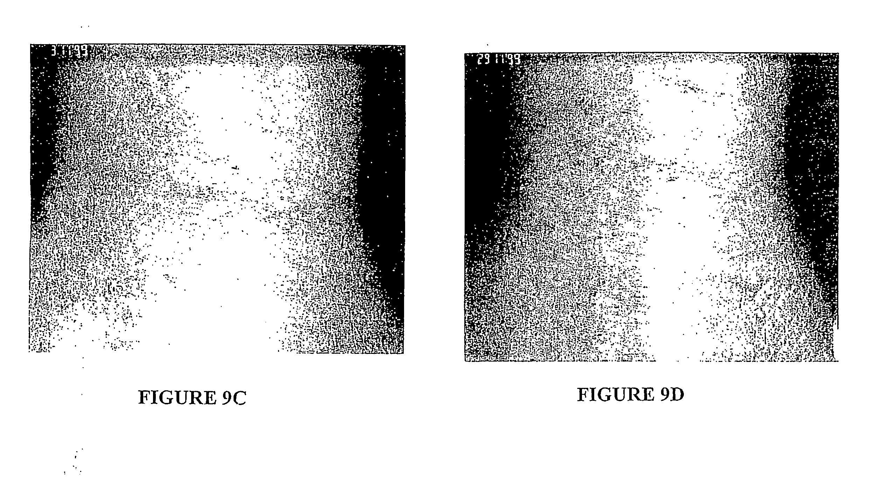

**Figures 9B to 9D** shows the improving condition of patient 4's wrist over approximately 7 weeks of taking the dosage as described in this invention. More particularly, figures 9B is taken 9 days after taking the prescribed dosage where the spots and inflammation have gone and the skin is returning to a smooth unblemished appearance. Similarly figure 9C shows the skin retaining the smooth unblemished appearance.
**Figure 9D** again shows the same wrist however there are now healing scabs along the flexural regions between the hand and wrist,

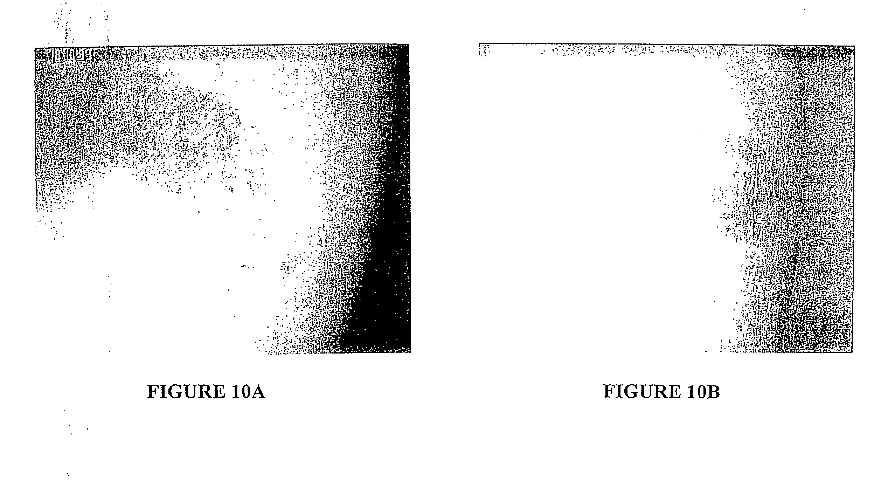

**Figure 10A** shows the condition of patient 4's flexural region on the arm before taking the liquid dosage as described in this invention. The eczema has caused inflammation and. swelling of the skin along the flexural lines with small spots forming. The patient was then prescribed the liquid dosage as described in the invention at a dosage rate of 10mls, 3 times a day,
**Figure 10B** shows the condition of the flexural region 9 days after patient 4 has been taking the prescribed dosage. The skin where the eczema had affected is now healing with the inflammation and redness disappearing. The skin is now returning to a smooth unblemished appearance.

The oral administration for the treatment of eczema and/ or psoriasis can be in addition to any other medicament administered for such ailment whether administered orally, topically, parenterally, sublingually, etc.

In practice the use of the medicament of the invention will involve ideally oral self administration of effective quantities of cetyl myristate alone or more preferably as a mixture of both cetyl myristate and cetyl palmitate.

Preferably in any such mixture the cetyl myristate comprises at least about half of the mixture or the serial application on a weight to weight basis. It is envisaged that daily doses will vary depending on patient needs and may range from 1 to 20 capsules per day. A capsule ideally contains between 5 to 370 mg of the mixture or cetyl myristate.

Trials with a variety of patients reliant upon dosage forms of cetyl myristate alone have shown favourable responses insofar as relief from the symptoms of IBS and/or IBD are concerned. It has been found however that enhanced benefits occur where there is at least a small proportion of cetyl palmitate in addition to the cetyl myristate and it is to the use of one such ratio of these active ingredients that the following trial examples relate.

Examples of use follows. Each briefly describes the patient's condition before and after the stated treatment using dosage forms (ie; "of the invention'') each having about 350 mg of the mixture of cetyl myristate and cetyl palmitate. That mixture comprises by weight 95% cetyl myristate and 5% cetyl palmitate by weight manufactured by the process as disclosed in NZ Patent Specification No. 332959. In addition added excipients were present in the non gelatin two part capsule case.

### TRIAL EXAMPLES:

### • Patient 1 is male and 5 and 1/2 years old

Has suffered since birth from eczema with associated intense pruritus generally all over the body. Patient 1 had multiple use of allopathic and homeopathic treatments including steroids and was on 11 different medications including Elocon, and a hydrocortisone cream.

At the first appointment, patient 1 was provided with the liquid form of a dosage unit as described in this invention which was taken at the rate of 5mls 3 times a day.

Three weeks later, patient 1 showed marked improvement over the entire body and all itching and psoriasis had stopped.

At the conclusion of 6 weeks, the patient's skin was virtually normal and soft with all infected lesions totally healed.

Patient 1 maintains a daily dose of 5mls 3 times a day and is now symptom and conventional medication free.

### • Patient 2 is male and 3 years old.

Had eczema from head to toe with marked scratching apparent over the upper and lower limbs. The patient was unable to sleep due to the scratching and used a wide range of allopathic medical moisturisers and steroids to try and cure the eczema.

At the first appointment patient 2 was provided with the liquid form of a dosage unit as described in this invention which was taken at the rate of 5 mls 3 a day.

One month later, the itching was gone, patient 2 was sleeping through the night, and the skin was more normal although there was still some scratching evident.

Two months later as there had not been any more improvement the dose was increased to 10 mls 4 times a day. This resulted in a marked improvement all over the skin surface.

The patient's skin is now nearing normal and now uses only a mild emollient and Fucicort to treat local areas of infection in conjunction with the invention. No other medications are used.

### • Patient 3 is female and 3 years old.

Had eczema over the legs, arms and abdomen with very rough, dry sand paper like skin. Patient 3 since birth had used bath oil and steroid creams to no avail.

At the first appointment patient 3 was provided with the liquid form of a dosage unit as described in this invention which was taken at the rate of 5mls 3 times a day.

Two weeks later, the patient was seen again. There had been a marked improvement in the skin, especially in the extremity and abdomen regions. This dosage has now been reduced to 5ml once daily.

The skin is continuing to soften and there is no longer any psoriasis. Patient 3 no longer takes any other medication or creams apart from the invention.

### • Patient 4 is female and 6 ½ years of age.

This young girl has had chronic eczema since 18 months of age and has been associated with very dry cracking skin associated with intense psoriasis. Her mother has used extensive amounts of hydrocortisone, Elecon (another steroidal cream) and BK Lotion, all of which managed to contain the eczema in local patches. However, the skin has continued to be dry and itchy.

At the first appointment Patient 4 was provided with the liquid form of a dosage unit as described in this invention which was taken at a rate of 10ml, 3 times a day. She was assessed in approximately one week and showed marked improvement, where her skin was less dry and the psoriasis or itch had improved.

Patient 4 continued with the invention over the next three months and continued improvement. Where her skin returned to normal with a lovely soft texture and the natural oils or sebaceous secretions were sufficient to moisturise her skin naturally. She has now used the medication for two years with no relapses.

### • Patient 5 is female and 7 years old.

Had severe eczema at all flexural surfaces i.e knees, elbows, wrists and was on a medication of potent steroids including Dermol and Eumovate cream.

At the first appointment patient 5 was provided with the liquid form of a dosage unit as described in this invention which was taken at the rate of 10mls 2 times a day which was mixed with honey.

Two and a half weeks later, the skin was beginning to return to normal on all flexural surfaces. This skin continued to improve and now patient 5 is symptom free and no longer taking the steroids. The patient has continued on with the invention.

### • Patient 6 is male and 28 years old.

Had eczema over the entire body with associated intense psoriasis with roughened skin over the abdomen and back. He also suffered an intense itch at multiple small cracks in flexural regions of the body and was unable to participate in sport as the perspiration entering the cracks caused intense stinging and pain.

At the first appointment patient 6 was provided with capsules of a dosage unit as described in this invention which were taken in groupings of 4 capsules 3 times a day.

Three weeks later, the skin had improved in texture, the itching and the dryness had gone, and the number of cracks at the flexural areas had decreased.

The patient has suffered a relapse after 6 weeks, when the patient discontinued with the invention.

But upon resuming with the programme proscribed with the invention the patients skin returned to its former state and the patient now enjoys normal skin. He has been taking the invention for over a year now.

### • Patient 7 is female and 3 years of age.

Patient 7 at age two was first seen with chronic eczema. Previous treatment included various bath oils, and a variety of hydrocortisone moisturising creams gave minimal improvement, and the eczema remained extensively all over her body.

Patient 7 was provided with the liquid form of a dosage unit as described in this invention which was taken at a rate of 5ml, 3 times a day

Patient 7 was reviewed after one week and showed a marked improvement. She has since continued with the invention and is on a maintenance dose of 5ml once a day. She has used this medication over the last year, and has gone for periods of about six months without using the invention as described, however during the winter, she goes back on the invention again. She no longer takes any other medication.

### • Patient 8 is male and 6 months old.

Patient 8 was seen first on 1 November 2000 with a history of eczema since birth. The eczema covered his entire body, over arms, abdomen and face. The mother had been using various natural remedies and moisturising creams, such as Alpha Keri lotion and bath oils. However, he was very unsettled because of the psoriasis.

At the first appointment he was provided with the liquid form of a dosage unit as described in this invention which was taken at a rate of 5ml 3 times a day. He was reviewed in one week's time where there was a marked improvement.

The skin on his abdomen, legs and face have now completely returned to normal. He now continues to do well on a maintenance dose of 5ml, twice a day.

### • Patient 9 is female and is 5 years of age.

Patient 9 has had eczema all her life. She does not get a full night's sleep because of the itch of psoriasis and has had recurrent infections. This has caused severe stress in her family. The mother had used all forms of steroid cream and had taken her daughter to naturopaths and homeopaths. All medications and potions prescribed did not alleviate the eczema and or psoriasis. This patient further has multiple allergies and is on a very strict diet.

At the first appointment she was provided with the liquid form of a dosage unit as described in this invention which was taken at a rate of 7.5ml, 3 times a day. She was reviewed in a week. There was some reduction in the psoriasis or itch, and the skin texture had improved slightly. The dosage was then increased to 10ml, three times a day, and she was review in two weeks' time.

Her condition had improved sufficiently in that she was now getting a good night's sleep and her skin although improved in texture, had not got to a soft delicate feeling yet. However, due to the increase in sleep and less itching she was a much brighter, happier child.

### • Patient 10 is female and 37 years of age.

Patient 10 has had severe chronic eczema involving her entire body, particularly her lower limbs. Patient 10 was only tried alternative medications including different supplements of Vitamin E, UPA oil and various lotions and lubricating creams to reduce the itch and problem with her skin.

At the first appointment Patient 10 was provided with capsules of a dosage unit as described in this invention which were taken in groupings of 4 capsules, 3 times daily. She has now been taking this dosage for approximately one year. She has reduced the dosage to 3 capsules, 3 times a day.

Patient 10 has lost all the itch and psoriasis that is associated with eczema and the texture in her skin, and particularly her lower legs have dramatically improved.

### • Patient 11 is male and 41 years of age.

Patient 11 has severe chronic eczema associated with his face, arms, legs and abdomen. He was also very disturbed by the psoriasis or constant itch that was associated with his condition. He has been treated by dermatologists and has used high potency steroid creams, lubricating creams, including Aquacare and Lipobase.

At the first appointment Patient 11 was provided with capsules of a dosage unit as described in this invention which were taken in groupings of 4 capsules, 3 times daily.

He was assessed after one week where his psoriasis had now improved and he was able to sleeping through the night without having to scratch. He was assessed again after taking the prescribed dosage after a further 3 months and his skin has become soft and the psoriasis has completely cleared up.

He has discovered that if he stops taking the invention the rash and eczema return in approximately four days of stopping the treatment.

As illustrated in these figures it can be plainly seen the improvement in overall skin condition when a patient is prescribed the dosage unit as described in the invention. The intensity of the inflammation and the reduction in scaliness of the skin, as illustrated in the colour copies, is reduced. This can be seen in figures 2, 6, 8, 9 and 10.

Further the lesions produced by the eczema and psoriasis is further reduced. This is seen in figures 1,2,3,5,6 and 7 wherein figure 5D it can be seen the skin has virtually cleared up.

## Claims

1. The use of (a)cetyl myristate or (b)cetyl myristate and cetyl palmitate in the manufacture of a medicament for the treatment and/or prophylaxis of eczema and/or psoriasis in a mammal.

2. A use as claimed in claim 1 wherein said medicament manufactured from (b) is for oral administration whether as a mixture of both cetyl myristate and cetyl palmitate, or serially.

3. A use as claimed in claim 2 wherein said administration is with a mixture of cetyl myristate in conjunction with cetyl palmitate where the cetyl myristate comprises from 50 to 98% w/w of the mixture.

4. A use of any one of the preceding claims wherein an effective amount of (a) or (b) is administered by means of one or more capsules.

5. A use of any one of the preceding claims where said mammal is a human being suffering from eczema or psoriasis and the administration is for treatment purposes.

6. A use of any one of the preceding claims where both cetyl myristate and cetyl palmitate in admixture are administered, the ratio by weight to weight being 95:5 respectively.

## Patentansprüche

1. Verwendung von (a) Cetylmyristat oder (b) Cetylmyristat und Cetylpalmitat, zur Herstellung eines Medikaments für die Behandlung und/oder Prophylaxe von Ekzem und/oder Psoriasis bei einem Säuger.

2. Verwendung nach Anspruch 1, worin das aus (b) hergestellte Medikament zur oralen Verabreichung von Cetylmyristat und Cetylpalmitat, entweder als Gemisch oder nacheinander, dient.

3. Verfahren nach Anspruch 2, worin die Verabreichung mit einem Gemisch von Cetylmyristat in Verbindung mit Cetylpalmitat erfolgt, wobei das Cetylmyristat von 50 bis 98% w/w des Gemischs umfasst.

4. Verwendung nach einem der vorhergehenden Ansprüche, worin eine effektive Menge von (a) oder (b) über eine oder mehrere Kapseln verabreicht wird.

5. Verwendung nach einem der vorhergehenden Ansprüche, worin der Säuger ein Mensch ist, der an Ekzem oder Psoriasis leidet, und die Verabreichung zu Behandlungszwecken erfolgt.

6. Verwendung nach einem der vorhergehenden Ansprüche, worin Cetylmyristat und Cetylpalmitat im Gemisch verabreicht werden, wobei das Gewichtsverhältnis 95 : 5 beträgt.

## Revendications

1. Utilisation (a) de myristate de cétyle ou (b) de myristate de cétyle et de palmitate de cétyle, dans la production d'un médicament destiné au traitement et/ou à la prophylaxie de l'eczéma et/ou du psoriasis chez un mammifère.

2. Utilisation suivant la revendication 1, dans laquelle ledit médicament produit à partir de (b) est destiné à l'administration orale, qu'il soit sous forme d'un mélange de myristate de cétyle et de palmitate de cétyle, ou qu'il soit destiné à l'administration en série de ces constituants.

3. Utilisation suivant la revendication 2, dans laquelle ladite administration est effectuée avec un mélange de myristate de cétyle et de palmitate de cétyle dans lequel le myristate de cétyle représente 50 à 98 % en poids/poids du mélange.

4. Utilisation suivant l'une quelconque des revendications précédentes, dans laquelle une quantité efficace de (a) ou (b) est administrée au moyen d'une ou plusieurs capsules.

5. Utilisation suivant l'une quelconque des revendications précédentes, dans laquelle ledit mammifère est un être humain souffrant d'eczéma ou de psoriasis, et l'administration est destinée à des fins de traitement.

6. Utilisation suivant l'une quelconque des revendications précédentes, dans laquelle le myristate de cétyle et le palmitate de cétyle sous forme d'un mélange sont administrés, le rapport en poids:poids, respectivement, étant de 95:5.
